# EUROPEAN PATENT SPECIFICATION

(11) **EP 3 323 420 B1**
(45) Date of publication and mention of the grant of the patent: **25.12.2019**
(21) Application number: 17179181.7
(22) Date of filing: 30.06.2017
(51) Int. Cl.: A61K 36/752, A61K 36/9066, A61K 31/122, A61K 36/068, A61K 36/67, A61K 45/06, A61P 39/00

(54) **DIETARY SUPPLEMENT AS AN ADJUVANT IN CHEMOTHERAPY AND RADIOTHERAPY**
NAHRUNGSERGÄNZUNGSMITTEL ALS ZUSATZ ZUR CHEMOTHERAPIE UND STRAHLENTHERAPIE
COMPLÉMENT ALIMENTAIRE COMME ADJUVANT À LA CHIMIOTHÉRAPIE ET LA RADIOTHÉRAPIE

(30) Priority: 18.11.2016 IT 201600116947
(43) Date of publication of application: 23.05.2018
(73) Proprietor: Idroflu S.r.l., 31015 Conegliano (TV) (IT)
(72) Inventor: Cosentino, Vincenzo, 31015 Conegliano TV (IT)
(74) Representative: Perani & Partners S.p.A.

(56) References cited:
- WO-A1-2013/054267
- WO-A1-2014/111268
- CN-A- 103 977 276
- DE-U1-202013 002 760

## Description

### FIELD OF THE INVENTION

The present patent application describes an association of active ingredients of natural origin and the formulations in which it is contained, for use as supplements in association with traditional anti-tumour therapies.

### STATE OF THE ART

In medicine, the term "antineoplastic chemotherapy" means all chemical substances that interfere directly or indirectly with cellular DNA replication mechanisms, capable of reducing proliferation and survival of tumour cells, and of normal cells, rapidly or constantly proliferating (bone marrow cells, mucous membranes, hair root, hair follicles).

Although they are not selective, chemotherapy drugs act on specific targets, eliciting their biologic effect by direct interaction with the DNA, which is damaged in a more or less reversible manner (alkylating agents, Platinum and its derivatives, anti-tumour antibiotics), or by indirect interaction with the nucleotide double helix, interfering with the synthesis of metabolites necessary for DNA replication (antimetabolites), or else interacting with the formation of the mitotic spindle and of the cell cytoskeleton (Vinca alkaloids, Taxanes). http://www.iss.it/lgac/docu/cont.php?id=301&tipo=32

Acting on all dividing cells, antineoplastic drugs do not affect specifically only cancer cells, but indistinctly all cells in the body, thus exhibiting high toxicity with respect to healthy cells undergoing replication. Fortunately, the ability to recover from the toxicity of antiblastic drugs is markedly higher in healthy cell systems than in tumour systems, in that tumour cells are unable to block the cell cycle or to activate the apoptosis mechanism in response to DNA damage, thus being more sensitive to chemotherapy agents and to their cytotoxic activity.

In combination or as an alternative to chemotherapy, antineoplastic radiotherapy is a particular type of anti-tumour physical therapy that uses ionizing radiation, generally X-rays, in the treatment of neoplasms.

The radiation, directed against the tumour mass with higher precision than in chemotherapy treatments, alters the biologic mechanisms that pertain to cell proliferation, inhibiting the growth of the tumour and promoting its progressive reduction.

In addition to direct cytotoxicity, intrinsic to the mechanism of action of radio and chemotherapeutic treatments, classic antineoplastic therapies are responsible for a decrease in the plasmatic levels of physiological antioxidants, indicating likely damage to the antioxidating defense mechanisms against the oxidative damage induced by the more common antiblastic therapies.

It is generally known that adequate levels of antioxidants are an indication of good health. An imbalance of the physiological cell equilibrium between the levels of oxidants and antioxidants, before, during or after the chemo/radiotherapeutic treatment, could be responsible for cell damage that lead, over the medium or long term, to the appearance of second generation tumours (1).

Substances of natural origin, known to be characterized by a good safety and toxicity profile, are repositories of active ingredients with great potential for the development of new antiblastic treatments or preventive therapies.

Nutrition, as a way to consume beneficial substances, or to associate natural adjuvants with the diet, is the new frontier in preventive or curative anti-tumour treatment. Numerous experimental results demonstrate that substances normally consumed with nutrition (e.g. Curcumin, Resveratrol, Epigallocatechin gallate, Vitamin A and precursors, isothiocyanates, sulforaphane, flavonoids) have a therapeutic effect against highly cancerous, treatment-resistant tumour cell populations (2).

In the journal Seminars and Cancer Biology, N.S. Yarla et al. give an overview (as of 2015) of the substances that are currently known to have preventive effects with respect to carcinogenesis, and in particular of those substances that exhibit an inhibitory effect with respect to the metabolic pathway of arachidonic acid, involved in tumour genesis. Apigenin, anthocyanins, baicalein, berberin, curcumin, allyl disulfide, ellagic acid, thymoquinone, ursolic acid, eugenol, fisetin, genistein, piperine, quercetin are only some of the active agents that have been demonstrated to inhibit the metabolic pathway of arachidonic acid and that are under investigation for the production of preventive agents against the onset of cancer (3).

A. Sahebkar et al., through a meta-analysis of randomized clinical tests, suggest that curcumin may have an important role in the reduction of the plasmatic levels of TNF-α (tumour necrosis factor-α) in patients suffering from depression, solid tumours, osteoarthritis, pulmonary complications, end-stage renal disease, and metabolic syndrome, where TNF-α is one of the most important cytokines in the mediation of chronic inflammation, a process that is closely related to the onset of chronic diseases of the heart, lungs, and of the immune system, and in the onset of cancer (4).

A widely used category of beneficial natural substances, both from the nutraceutical and the diet point of view, are carotenoids, natural pigments known to the public at large for their antioxidant and anti-aging properties exhibited in vitro and in animal models (5), (6). Among the different types of carotenoids, those isolated from marine plants have proved to be particularly effective in inhibiting the vitality of different types of tumour cells (breast, intestine, liver, bladder, prostate, oral mucosa tumour and in case of leukaemia), and they have exhibited a beneficial anti-obesity and antiinflammatory, as well as cardioprotective, effect (7).

The intake of astaxanthin through the diet (40µg/kg of body weight/day) in syngeneic mice inoculated with low doses of methylcholanthrene (Meth-A)-induced fibrosarcoma tumour cells, has revealed a reduction in tumour growth relative to the control, both in terms of weight and of size. This therapeutic effect was observed when the astaxanthin supplemented diet had been started from 1 and 3 weeks before inoculation of the Meth-A tumour cells. When higher tumour doses were administered to the syngeneic mice, the beneficial effect of astaxanthin could not be detected, suggesting a potential carcinogenic prevention effect of the carotenoid limited to the initial stages of development of the cancer (8).

Among the new resources for the improvement of patients' survival rates are also included some medicinal mushrooms, which have, first of all, an effect of stimulating the immune system, often significantly damaged by classic antineoplastic therapies, such as radiotherapy and chemotherapy. The mushrooms Agaricus blazei, Ganoderma lucidum, Cordyceps sinensis, Trametes versicolor, Grifolia frondosa, when tested *in vitro, in vivo* (on animal models) and *ex vivo,* have proved to stimulate a beneficial immune response, increasing the set of cytokines correlated thereto (9).

### SUMMARY OF THE INVENTION

The Applicant has now surprisingly found that the association of astaxanthin, curcumin and dry extract of Cordyceps sinensis exhibits a synergic effect promoting cell apoptosis in radiotherapeutic or chemotherapeutic treatments, eliciting the cytotoxic effect in different ways with respect to the aforementioned classic antiblastic therapies.

This association minimizes the oxidative stress induced by the antineoplastic therapy, and activates proapoptotic factors, improving the effectiveness of chemotherapeutic and radiotherapeutic treatments, without the side effects of the aforesaid therapies.

Therefore, the object of the present invention is a nutraceutical association comprising astaxanthin, dry extract of Cordyceps sinensis and curcumin.

A further object of the present invention further is a formulation comprising a nutraceutical association comprising astaxanthin, dry extract of Cordyceps sinensis and curcumin, in combination with suitable excipients and/or diluents.

### DESCRIPTION OF THE FIGURES

Figure 1: Plasmatic levels of PSA in the SM plus group patients relative to the SM group patients, measured at the end of the treatment period (6 weeks), compared to those measured at the beginning of the study (T = 0).
Figure 2: Plasmatic levels of free radicals in the SM plus group patients relative to the SM group patients, measured at the end of the treatment period (6 weeks), compared to those measured at the beginning of the study (T = 0).

### DETAILED DESCRIPTION OF THE INVENTION

The object of the present invention is a pharmaceutical or nutraceutical association comprising astaxanthin, curcumin and dry extract of Cordyceps sinensis.

For the purposes of the present invention, "association" means the combination of two or more active ingredients, which act together for the achievement of a single synergic effect.

The term "extract" means a concentrated pharmaceutical preparation, comprising one or more molecules, obtained by extracting medicinal substances of interest from a biological matrix of origin. An extract is considered dry when it has a dry residue of no less than 95% by mass.

Cordyceps sinensis is a parasitic fungus of a lepidopteran of the Hepialidae family, whose larva buried in the ground it progressively invades, and from which cordycepin is extracted.

Said medicinal fungus is extremely rich in beta-glucans, recognized as aids in therapies against cancer, and in polysaccharides, long sugar chains with numerous oxygen sections within them. When they are metabolized and broken down by the body to their constituent units, polysaccharides release oxygen molecules which are absorbed by the cells, exhibiting a proapoptotic adjuvant action, considering that neoplastic cells do not survive in an oxygen-rich environment.

The dry extract of Cordyceps sinensis contained in the association of the present invention is preferably a dry extract with an extraction ratio between 4:1 and 8:1 in ethanol, where the term "extraction ratio" means the ratio between the units by weight of fresh material to be extracted and the units by weight of dry extract obtained from the aforesaid extraction.

Yet more preferably, the dry extract of Cordyceps sinensis has an extraction ratio in ethanol of 4:1.

The curcumin contained in the association of the present invention is preferably in the form of dry extract from Curcuma longa rhizome with 95% curcumin titre.

Astaxanthin is preferably in the form of oleoresin with 95% total astaxanthins or as 2.5% adsorbed powder of the oleoresin extracted from algae in O2 supercritical flow.

Yet more preferably, the association subject of the present invention comprises astaxanthin and 4:1 - 8:1 dry extract of Cordyceps sinensis in a weight ratio between 1:5 and 1:500, astaxanthin and curcumin in a weight ratio between 1:5 and 1:500.

More preferably, astaxanthin and 4:1 - 8:1 Cordyceps sinensis dry extract are in a weight ratio between 1:40 and 1:80, while astaxanthin and curcumin are in a weight ratio between 1:10 and 1:50.

Advantageously, when combined in specific weight ratios, astaxanthin, dry extract of Cordyceps sinensis and curcumin are effective in preventing the side effects of radio and chemotherapy and in enhancing the cytotoxic activity of said therapies.

The association subject of the present invention promotes cell apoptosis, in different ways with respect to the chemo-radiotherapeutic treatment, while minimizing the oxidative stress induced by these antiblastic therapies.

Surprisingly, when associated, astaxanthin, dry extract of Cordyceps sinensis and curcumin act synergistically to obtain the aforementioned effects, exhibiting greater effectiveness than that characterizing the three individual substances.

Cordycepin (or 3'-deoxyadenosine), an active substance contained in the extract of Cordyceps sinensis, is a derivative of the adenosine nucleoside, differing therefrom by the absence of an oxygen atom in the position 3 of the ribose ring.

Cordycepin increases the activation of pro-apoptosis proteins, by a bond with the DR3 receptor and consequently by the activation of caspase 3-8 (10). At the same time, cordycepin seems to have an anti-metastatic action as well, by stimulation of the A₃ adenosine receptor and by inhibition of the matrix metalloproteinase-2 and -9, involved in the migration of tumour cells (11, 12).

Astaxanthin is a naturally occurring red pigment present in numerous living organisms. However, the main extractive source is vegetable, in that it is exclusively synthesized from the unicellular microalga Haematococcus pluvialis as a photoprotective substance for its own cells and spores.

Astaxanthin promotes oxygen transport by enhancing blood flow, making cells more sensitive to chemotherapy and radiotherapy, reduces the cytotoxicity of the chemotherapeutic treatment with mitomycin C, by reducing the expression of RAD51, a gene coding proteins tasked with repairing the DNA double helix, and by activating the protein kinase B (AKT), involved in the signalling pathway that regulates cell survival and death (13, 14). Astaxanthin also inhibits the activator of transcription 3 (STAT3), promoter of tumour cell proliferation (14).

Curcumin is the main biologically active component of Turmeric (also known as *Curcuma longa*)*,* a component of the botanic group of Curcuma, belonging to the ginger family.

Curcumin is able to reduce the concentration of Tumour Necrosis Factor Alpha (TNFα), a cytokine involved in systemic inflammation, in the acute phase (4); it also inhibits the Nuclear Factor NFKB which is activated in various forms of neoplasm.

Without being bound to any theory, it is believed that curcumin, in the association of the present invention, elicits its biological effect through the regulation of the expression of inflammatory cytokines.

The association subject of the present patent application is preferably employed for the preparation of dietary supplements, preferably employed as adjuvants in chemotherapy and radiotherapy.

The term "dietary supplements" means specific products directed at promoting the intake of determined active ingredients if the body needs them or in the presence of an incorrect diet.

The term "adjuvant" means a substance or a set of biologically active substances to be taken in combination with classic antiblastic therapies, to enhance their therapeutic effects.

Still more preferably, the association according to the present invention is employed as an adjuvant in intravesical chemotherapy.

The association, when employed as an adjuvant in chemotherapy and radiotherapy and preferably in intravesical chemotherapy, can be administered before starting the therapies, during the treatments and/or after the end of the treatments.

Still more preferably, the association is employed to reduce the irritative symptomatology of post radiotherapy actinic cystitis and of post intravesical chemotherapy chemical cystitis.

The greatest problem of bladder cancer is the recurrence of the disease. Within one year from the first endoscopic surgery (TURBK), approximately 20% of Patients with low-grade neoplasm, and 40% of Patients with high-grade disease, develop a recurrence of the disease.

For this reason, after surgery, a cycle of intravesical chemotherapy by local drug instillations is often prescribed. The procedure is carried out using a small catheter, emptying the urinary bladder and introducing an intravesical drug, which the patient is asked to retain in the bladder for approximately one hour, with side effects mainly related to the irritation of the local mucosa. http://www.hsr.it/clinica/specialita-cliniche/urologia/urologia-san-raffaele/patologie-e-trattamenti/chemioterapia-endovescicale/

Actinic cystitis is an inflammation of the bladder wall, resulting from pelvic radiotherapy, hence radiotherapy carried out for prostate, cervix, bladder and colorectal tumours.

Approximately 20% of patients treated in this region develop cystitis symptoms, of different severity. Of these, approximately 10% have severe cases, even with abundant presence of blood in the urine. The cause of the onset of the disease is that the rays may also affect tissue neighbouring the target organ, causing ulcerations and inflammation.

In particular, damage occurs to the innermost layer of glycosaminoglycans of the bladder, which is a breach in the integrity of the parenchyma, allowing harmful substances to pass through it. http://www.cistite.eu/tag/cistite-da-radioterapia/

According to a preferred embodiment, the neutraceutical association described herein is in the form of a formulation comprising astaxanthin in a quantity that varies between 0.1% and 5% by weight over the total weight of the formulation, 4:1 - 8:1 dry extract of Cordyceps sinensis in amounts between 10% and 30% by weight over the total weight of the formulation, curcumin in amounts between 8% and 30% by weight over the total weight of the formulation and suitable excipients.

Said formulation may contain, in addition to the association, additional active ingredients beneficial for the reduction of the side effects from antiblastic drugs or for the enhancement of their therapeutic effect.

In the preferred embodiment, the formulation carrying the aforementioned association is prepared according to the teachings disclosed in the EP patent application EP2849731.

Yet more preferably, the formulation carrying the association subject of the invention further comprises a combination of: a polysorbate, a chitosan salt with N-acetylcysteine, an extract of Grapefruit seed polyphenols with 95% titre and Piperine extracted from Piper Nigrum with 95% titre.

More preferably, the polysorbate is selected from one of the commercially available polysorbates, e.g. liquid polysorbates 20 and 80.

The formulation thus obtained is preferably produced in the form of an enteric-coated tablet, in which a single dosage unit preferably contains astaxanthin between 0.1% and 5%, 4:1 - 8:1 dry extract of Cordyceps sinensis between 10% and 30% and curcumin between 8% and 30%.

Preferably, the association in the form of a formulation comprising astaxanthin in an amount varying between 0.1% and 5% by weight over the total weight of the formulation, 4:1 - 8:1 dry extract of Cordyceps sinensis in amounts between 10% and 30% by weight over the total weight of the formulation, curcumin in amounts between 8% and 30% by weight over the total weight of the formulation and suitable excipients, is administered at least twice a day.

A further object of the present invention is a formulation comprising an association comprising astaxanthin, curcumin and dry extract of Cordyceps sinensis, in combination with suitable excipients and/or diluents.

### EXAMPLES

### Experimental Data

The Applicant has carried out an observational clinical study (registry, supplement study) directed at evaluating the therapeutic activity of the nutraceutical association subject of the application, within the scope of the integrated cancer therapy.

Integrated cancer therapy clinical trials ("Supplement human studies") define the field of activity of supplements in support to antiblastic drug therapy and their potential pre-therapeutic, preventive applications.

Said integrated cancer therapy clinical trials produce supplementary data that must be compared to those obtained from standard cancer treatment plans.

Since they entail the use of substances with an excellent safety profile and that meet pharmaceutical standards, supplement human studies do not strictly require the approval of an ethics committee (15 - 17).

### Methods and patient selection

The study was carried out in patients suffering from genitourinary cancer (prostate or bladder malignancies) who underwent and completed cancer treatments (radiotherapy, chemotherapy or intravesical immunotherapy) at least one week before the start of the study.

The candidates were selected applying the following inclusion criteria:
- subjects aged between 55 and 75 years, suffering from prostate cancer (T1/T2) or bladder cancer;
- subjects who have completed a treatment (radiotherapy, chemotherapy or intravesical immunotherapy), at least one week before the start of the study.

In addition, the following exclusion criteria were applied:
- subjects with thrombosis or infections;
- subjects treated or undergoing therapy with drugs for other chronic or significant disorders;
- subjects who are allergic to drugs, or to foods, related to the products being investigated.

Of the 61 informed participants, 35 freely decided to follow a standard "SM" therapeutic plan for cancer treatment (control group), comprising radio or chemotherapeutic treatment with instillation, by means of validated protocols, recognized nationally and internationally; 26 subjects decided to follow an "SM plus" integrated cancer therapy program (supplement group) where, following the treatment with the SM standard therapeutic plan, a daily administration of 2 tablets per day of the nutraceutical association under investigation was followed, for at least 6 weeks.

The patients participating in the study were treated with an oral formulation comprising the nutraceutical association subject of the invention, whose dosage/units (e.g., tablet) are shown in the following Table 1.

The following clinical evaluations and laboratory tests were carried out at the beginning of the study (T = 0) and at the end of the treatment period (T = 6 weeks):
- evaluation of the severity of the side effects related to the treatment, by means of an arbitrary analog scale that quantifies the severity of the symptom with a numeric score. A score of 0 indicates the absence of side effects; a score of 5 indicates the presence of very severe side effects requiring medical attention;
- complete blood count;
- evaluation of the prostate-specific antigen (PSA);
- evaluation of free radicals in the blood: oxidative stress was evaluated by measuring the derivatives of reactive oxygen metabolites (d-ROMs), using a free radical analytical system (FRAS).

Acting on all dividing cells, antineoplastic drugs do not affect specifically only cancer cells, but indistinctly all cells in the body. Blood count monitoring makes it possible to evaluate the vitality of blood cells when subjected to standard cancer treatment (SM) or to integrated cancer therapy (SM plus).

PSA (prostate-specific antigen) is a protein produced by the prostate. Its dosage in the blood is measured because an increase thereof may indicate the presence of a prostate tumour. PSA levels below 4 ng/mL are generally considered normal.

However, the detection of high values is not sufficient to formulate a tumour diagnosis, because the PSA value may also rise in case of inflammatory prostate diseases, such as prostatitis, or in case of marked hypertrophy (or prostate hyperplasia or adenoma, i.e. an enlargement of the central portion of the prostate).

The d-ROMs Test (Diacron International) is a photometric test that allows to determine, in a biological sample, the concentration of reactive oxygen metabolites (ROM) and in particular of the hydroperoxides (ROOH), generated in cells by the oxidative attack of reactive oxygen species (ROS) on several biochemical substrates (lipids, amino acids, peptides, proteins, nucleotides, etc.). ROM concentration is expressed in CARR Units, from the last name of the researcher (Carratelli) who invented, patented and developed the d-ROMs Test, and the principle on which it is based is the Fenton reaction (http://www.diacron.com/index.php/it/products/kits-carratelli-panel/d-roms-test)

### Statistical analysis

Intragroup comparisons were carried out with Student's t-test. A value *p* < 0.05 was considered significant from a statistical viewpoint.

### Results

A total of 61 patients suffering from genitourinary cancer, previously treated with standard antiblastic therapies, were recruited.

Of the 61 recruited patients, two withdrew during the study period, thereby reducing the total group of volunteers to 59 participants, of which 26 in the supplement group and 33 in the control group.

The age of the patients included in the study varies between 58 and 74 years.

The distribution of the cancer type within the group of patients included in the study, as well as the related treatment, are shown in Table 2.

**Table 2: Interval of the analogue arbitrary scale: 0 (no side effect) - 5 (severe side effects requiring medical attention). The data are expressed as mean ± standard deviation. (* p<0.05 vs. T=0).**

| **Genitourinary cancer and treatment** | **SM (standard treatment program)** | **SM plus (standard treatment program + nutraceutical association)** |
|---|---|---|
| **Prostate cancer** | | |
| Chemotherapy | 8 | 6 |
| Radiotherapy | 7 | 4 |

| **Bladder cancer** | | |
|---|---|---|
| Chemotherapy | 8 | 6 |
| Radiotherapy | 5 | 5 |
| Intravesical immunotherapy | 6 | 5 |
| **TOTAL** | **33** | **26** |

Table 3 summarizes the severity of the side effects related to the treatment, determined by comparison with an arbitrary analogue scale.

**Table 3: The data are expressed as mean± standard deviation. P < 0.05 vs. T=0**

| | **SM (standard treatment program)** | | **SM plus (standard treatment program + nutraceutical association)** | |
|---|---|---|---|---|
| | T = 0 | 6 weeks | T = 0 | 6 weeks |

| **Symptoms** | | | | |
|---|---|---|---|---|
| Pain | 4.3±0.7 | 3.3±1.1 | 4.6±0.2 | 3.1±1.0* |
| Dysuria | 3.2±1.2 | 3.0±1.1 | 3.8±1.1 | 2.1±0.7* |
| Urgency | 3.4±0.4 | 3.1±0.6 | 3.7±1.2 | 2.0±1.0* |
| Infections | 3.4±0.3 | 2.5±0.7 | 3.3±0.5 | 1.2±0.3* |
| Nausea/vomiting | 2.3±0.5 | 2.0±0.2 | 2.2±0.4 | 1.0±0.5* |
| Tiredness/fatigue | 3.4±0.8 | 3.0±0.9 | 3.5±1.0 | 2.1±0.7* |

| **Signs** | | | | |
|---|---|---|---|---|
| Haematuria | 3.0±0.3 | 2.4±0.6 | 3.1±0.4 | 2.0±0.5* |
| Retention | 3.7±0.8 | 2.8±0.7 | 3.6±1.0 | 1.8±0.4* |

In the examined patients, the intensity of signs and symptoms diminished significantly after the 6-week observational period.

No significant change was observed in the control group.

It was also observed that administration of the nutraceutical association is associated with a significant improvement in the haematological parameters.

With reference to Table 4, a significant improvement was observed in particular in haemoglobin levels and in the number of erythrocytes and leukocytes in the group of patients treated with SM plus.

**Tabella 4: The data are expressed as mean ± standard deviation (* p<0.05 vs. T=0).**

| | **SM (standard treatment program)** | | **SM plus (standard treatment program + nutraceutical association)** | |
|---|---|---|---|---|
| | T = 0 | 6 weeks | T = 0 | 6 weeks |
| Haemoglobin (g/dL) | 13.8±1.0 | 13.8±1.1 | 13.8±1.1 | 13.9±1.0* |
| Erythrocytes (10⁶/mm³) | 4.4±0.4 | 4.5±0.7* | 4.6±0.7 | 4.8±0.6* |
| Leukocytes (10³/mm³) | 4.3±1.0 | 4.3±0.5 | 4.3±0.8 | 4.8±0.5* |

The increase of the aforesaid values suggests that the intake of the nutraceutical association of the invention at the end of the antiblastic treatment enables greater recovery of cell vitality compared to subjects who were not administered the support therapy after the standard cancer treatment.

A significant increase in erythrocytes was also recorded in control group patients.

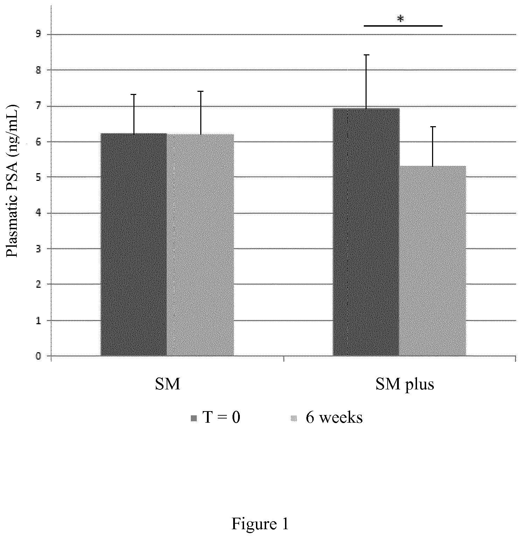

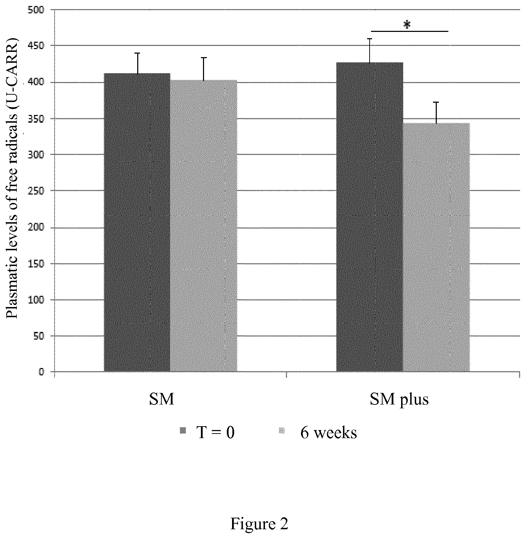

With reference to Figures 1 and 2, blood tests revealed a lowering of PSA and free radical levels in the patients of the SM plus group at the end of the treatment period, compared to the values measured at the start of the study (Figures 1 and 2).

### Safety

All patients took the supplement correctly, showing full observance of the treatment (100%) and compliance with the protocol (100%). No adverse effects or toxicity phenomena were reported.

### Conclusions

In the present observational study, a considerable improvement in the profile of side effects was observed in patients with genitourinary cancer (prostate and bladder tumours) who were administered a formulation containing the nutraceutical association of the present invention.

The observed clinical benefit is believed to be due to the biological properties of each of the ingredients comprised in the aforementioned association.

In particular, from the study it emerged that the nutraceutical association of the study has anti-oxidizing properties, reducing the free radical levels in patients with genitourinary cancer (344±28 vs 427±33U-CARR).

In spite of some limitations, such as the heterogeneity of the study population and the limited size of sample, this study has shown how a nutritional approach based on ingredients derived from natural substances can help the body to cope with the toxicity of genitourinary cancer treatment.

### EXAMPLE 1

For illustrative and non-limiting purposes, an example of embodiment of the formulation of the present invention is described below, in the form of a single dosage unit, therefore corresponding to half of the daily dose to be taken for use as an adjuvant in chemotherapy and radiotherapy.

**Table 1**

| Step | Ingredient | mg |
|---|---|---|
| A | Curcuma longa rhizome dry extract with 95% curcumin titre | 132 mg |
| A | Astaxanthin | 4.0 mg |
| A | 4:1 dry extract of Cordyceps sinensis in ethanol | 250 mg |
| B | N -acetyl cysteine | 5.0 mg |
| B | Chitosan | 2.0 mg |
| B | Polysorbate 80 | 50 mg |
| B | Water | q.s. dissolution |
| c | N-acetylcysteine | 100 mg |
| c | Chitosan | 30 mg |
| c | Piperine from black pepper dry extract 95% | 5 mg |
| c | Polyphenols from Grapefruit seed extract dry extract 95% | 50 mg |
| D | Calcium phosphate dibasic dihydrate E341 | 500 mg |
| D | Crosslinked sodium carboxymethyl cellulose E468 | 36 mg |
| D | Precipitated amorphous silica E551 | 15 mg |
| D | Magnesium stearate E470b | 15 mg |
| D | Pregelatinized corn starch | 50 mg |
| E | Shellac ammonium salt (SHELLAC WATER 25% E904-E503) | 150 mg |
| F | Clear Sepifilm LP030 | 40 mg |

### Preparation method:

***a)*** In a powder mixer, add under stirring the powder components of Step A at ambient temperature;
***b)*** In the minimum volume of demineralized water, dissolve the N-acetylcysteine and subsequently add the CHITOSAN under stirring until its complete dissolution (clear, slightly yellowish phase). Add the POLYSORBATE 80 under slow stirring to avoid the formation of foam. Add the aqueous solution of the Chitosan salt to what was obtained from step ***a*** under stirring, until obtaining a uniform granulate, without non-moistened parts. Dry in ventilated furnace at 45°C, until weight is constant. Sift the granulate.
*c)* Mix the powders of Step C and add the powder thus obtained with the powder obtained from step ***b*** until obtaining a mixture having an even particle size;
***d)*** Compress the powder obtained from step ***c*;**
***e)*** Coat the tablet obtained in step ***d*** with gastro-resistant coating, in a coating pan;
***f)*** Film coating of the tablet per step ***e***;

The enteric coated tablet has a final weight of approximately 1300 mg.

*TEST:* the tablet thus obtained was subjected to the assays of weight uniformity, hardness, friability and abrasion in accordance with the FUI (Italian Official Pharmacopoeia).

### BIBLIOGRAPHY:

1) Integrative Cancer Therapies 6(3); 2007; pp. 281- 292 DOI: 10.1177/1534735407305655; Do Antioxidants Interfere with radiation therapy for cancer? Ralph W. Moss, PhD
2) Mol Nutr Food Res. 2016 Apr 6. doi: 10.1002/mnfr.201500887; Targeting cancer stem-like cells using dietary-derived agents - where are we now? Khan S, Karmokar A, Howells L, Thomas AL, Bayliss R, Gescher A, Brown K.
3) Semin Cancer Biol. 2016 Feb 4. pii: S1044-579X(16)30003-7. doi: 10.1016/j.semcancer.2016.02.001. [Epub ahead of print]; Targeting arachidonic acid pathway by natural products for cancer prevention and therapy. Yarla NS, Bishayee A, Sethi G, Reddanna P, Kalle AM, Dhananjaya BL, Dowluru KS, Chintala R, Duddukuri GR.
4) Pharmacol Res. 2016 Mar 26;107:234-242. doi: 10.1016/j.phrs.2016.03.026. [Epub ahead of print]; Curcumin downregulates human tumour necrosis factor-α levels: A systematic review and meta-analysis ofrandomized controlled trials. Sahebkar A, Cicero AF, Simental-Mendia LE, Aggarwal BB, Gupta SC.
5) Int J Mol Sci. 2016 Jan 14;17(1). pii: E103. doi: 10.3390/ijms17010103. Free Radical Scavenging and Cellular Antioxidant Properties of Astaxanthin. Dose J, Matsugo S, Yokokawa H, Koshida Y, Okazaki S, Seidel U, Eggersdorfer M, Rimbach G, Esatbeyoglu T.
6) Journal of Agricultural and Food Chemistry, vol.61, 2013, pp.7800-7804. Antiaging Effects of Astaxanthin-Rich Alga Haematococcus pluvialis on Fruit Flies under Oxidative Stress. HUANGFU Jie Qiong, LIU Jin, ZHENG Sun, WANG Ming Fu, JIANG Yue, CHEN Zhenyu, CHEN Feng.
7) Crit Rev Food Sci Nutr. 2015 Nov 13:0. [Epub ahead of print]. Marine Carotenoids: Bioactivities and Potential Benefits to Human Health. Chuyen VH, Eun JB.
8) Nutr Cancer. 2000;36(1):59-65. Anti-tumour activity of astaxanthin and its mode of action. Jyonouchi H, Sun S, Iijima K, Gross MD.
9) Integr Med (Encinitas). 2014 Feb;13(1):32-44. Immune Modulation From Five Major Mushrooms: Application to Integrative Oncology. Guggenheim AG, Wright KM, Zwickey HL.
10) Oncol Lett. 2015 Aug; 10(2): 595-599. Published online 2015 May 27. doi: 10.3892/ol.2015.3273. Apoptosis and inhibition of proliferation of cancer cells induced by cordycepin. XUEWEN TIAN, YUJIAN LI, YINYU SHEN, QIAOQIAO LI, QINGLU WANG, and LIANSHI FENG
11) Journal of Pharmacological Sciences; Volume 127, Issue 1, January 2015, Pages 53-56. Anticancer and antimetastatic effects of cordycepin, an active component of Cordyceps sinensis. Kazuki Nakamura, Kazumasa Shinozuka, Noriko Yoshikawa
12) Int J Oncol. 2013 Mar;42(3):1036-44. doi: 10.3892/ijo.2013.1762. Epub 2013 Jan 4. Apoptosis induction of human prostate carcinoma cells by cordycepin through reactive oxygen species-mediated mitochondrial death pathway. Lee HH, Park C, Jeong JW, Kim MJ, Seo MJ, Kang BW, Park JU, Kim GY, Choi BT, Choi YH, Jeong YK.
13) Biochem Pharmacol. 2016 Apr 1;105:91-100. doi: 10.1016/j.bcp.2016.02.016. Epub 2016 Feb 24. Astaxanthin down-regulates Rad51 expression via inactivation of AKT kinase to enhance mitomycin C-induced cytotoxicity in human non-small cell lung cancer cells. Ko JC, Chen JC, Wang TJ, Zheng HY, Chen WC, Chang PY, Lin YW.
14)Mar Drugs. 2015 Jul 14;13(7):4310-30. doi: 10.3390/md13074310. Multiple Mechanisms of Anti-Cancer Effects Exerted by Astaxanthin. Zhang L, Wang H.
15) Belcaro G. Pharma Standard Supplements. Clinical applications. Imperial College Press, World Scientific Publications, London-Singapore, 2016.
16) Belcaro G, Cornelli U, Ledda A, Hosoi M. Assessment of nutraceuticals and food supplements. Panminerva Med 2011; 53: I-II.
17) Singh R, Wang O. Clinical trials in "emerging markets": regulatory considerations and other factors. Contemp Clin Trials 2013; 36: 711-8.

## Claims

1. A nutraceutical association comprising astaxanthin, curcumin and Cordyceps sinensis dry extract.

2. The association according to claim 1 wherein the Cordyceps sinensis dry extract has an extraction ratio between 4:1 and 8:1.

3. The association according to claim 2 wherein astaxanthin and Cordyceps sinensis 4:1 - 8:1 dry extract are in a weight ratio between 1:5 and 1:500, astaxanthin and curcumin are in a weight ratio between 1:5 and 1:500.

4. The association according to any one of the claims from 1 to 3 for use as a dietary supplement.

5. The association for use according to claim 4 as an adjuvant in chemotherapy and radiotherapy.

6. The association for use according to claim 5 as an adjuvant in intravesical chemotherapy.

7. The association for use according to claim 5, as an adjuvant for the treatment of actinic cystitis and chemical cystitis.

8. The association for use according to any of the claims 4 to 7, wherein the association is in the form of a formulation comprising astaxanthin between 0.1% and 5%, 4:1 - 8:1 dry extract of Cordyceps sinensis between 10% and 30%, curcumin between 8% and 30% by weight over the total weight of the formulation and suitable excipients.

9. The association for use according to claim 8, wherein said formulation further comprises a polysorbate, a chitosan salt with N-acetylcysteine, an extract of polyphenols from Grapefruit with 95% titre and Piperine extracted from Piper Nigrum with 95% titre.

10. The association for use according to any of the claims from 8 to 9, wherein said formulation is administered at least twice per day.

11. A formulation comprising a nutraceutical association comprising astaxanthin, curcumin and dry extract of Cordyceps sinensis, in combination with suitable excipients and/or diluents.

## Patentansprüche

1. Eine nutrazeutische Assoziation, die Astaxanthin-, Curcumin- und Cordyceps sinensis-Trockenextrakt umfasst.

2. Assoziation nach Anspruch 1, wobei der Cordyceps sinensis-Trockenextrakt ein Extraktionsverhältnis zwischen 4:1 und 8:1 aufweist.

3. Assoziation nach Anspruch 2, wobei Astaxanthin und Cordyceps sinensis-Trockenextrakt 4:1 - 8:1 in einem Gewichtsverhältnis zwischen 1:5 und 1:500 vorliegen und Astaxanthin und Curcumin in einem Gewichtsverhältnis zwischen 1:5 und 1:500 vorliegen.

4. Assoziation nach einem der Ansprüche 1 bis 3 zur Verwendung als Nahrungsergänzungsmittel.

5. Assoziation zur Verwendung nach Anspruch 4 als Adjuvans in der Chemotherapie und Strahlentherapie.

6. Assoziation zur Verwendung nach Anspruch 5 als Adjuvans in der intravesikalen Chemotherapie.

7. Assoziation zur Verwendung nach Anspruch 5 als Adjuvans zur Behandlung von aktinischer Zystitis und chemischer Zystitis.

8. Assoziation zur Verwendung nach einem der Ansprüche 4 bis 7, wobei die Assoziation in Form einer Formulierung vorliegt, die Astaxanthin zwischen 0,1% und 5%, Cordyceps sinensis-Trockenextrakt 4:1 - 8:1 zwischen 10% und 30%, Curcumin zwischen 8% und 30 Gew.-%, bezogen auf das Gesamtgewicht der Formulierung und geeigneter Trägerstoffe, umfasst.

9. Assoziation zur Verwendung nach Anspruch 8, wobei die Formulierung ferner ein Polysorbat, ein Chitosansalz mit N-Acetylcystein, einen Polyphenolextrakt aus Pampelmuse mit 95% Titer und aus Piper Nigrum mit 95% Titer extrahierte Piperin umfasst.

10. Assoziation zur Verwendung nach einem der Ansprüche 8 bis 9, wobei die Formulierung mindestens zweimal täglich verabreicht wird.

11. Formulierung umfassend eine nutrazeutische Assoziation umfassend Astaxanthin, Curcumin und Cordyceps sinensis-Trockenextrakt in Kombination mit geeigneten Exzipienten und/oder Verdünnungsmitteln.

## Revendications

1. Une association nutraceutique comprenant de l'astaxanthine, de la curcumine et de l'extrait sec de Cordyceps sinensis.

2. Association selon la revendication 1, dans laquelle l'extrait sec de Cordyceps sinensis a un rapport d'extraction compris entre 4:1 et 8:1.

3. Association selon la revendication 2, dans laquelle l'astaxanthine et l'extrait sec de Cordyceps sinensis 4:1 - 8:1 ont un rapport pondéral compris entre 1:5 et 1:500 et l'astaxanthine et la curcumine ont un rapport pondéral compris entre 1:5 et 1:500.

4. Association selon l'une quelconque des revendications 1 à 3 pour l'utilisation comme complément alimentaire.

5. Association pour l'utilisation selon la revendication 4 en tant qu'adjuvant en chimiothérapie et en radiothérapie.

6. Association pour l'utilisation selon la revendication 5 en tant qu'adjuvant en chimiothérapie intravésicale.

7. Association pour l'utilisation selon la revendication 5, en tant qu'adjuvant pour le traitement de la cystite actinique et de la cystite chimique.

8. Association pour l'utilisation selon l'une quelconque des revendications 4 à 7, dans laquelle l'association se présente sous la forme d'une formulation comprenant de l'astaxanthine entre 0,1% et 5%, de l'extrait sec de Cordyceps sinensis 4:1 à 8:1 entre 10% et 30%, de la curcumine entre 8% et 30% en poids par rapport au poids total de la formulation et des excipients appropriés.

9. Association pour l'utilisation selon la revendication 8, dans laquelle ladite formulation comprend en outre un polysorbate, un sel de chitosan avec de la N-acétylcystéine, un extrait de polyphénols de pamplemousse à 95% de titre et de la pipérine extraite de Piper Nigrum à 95% de titre.

10. Association pour l'utilisation selon l'une quelconque des revendications 8 à 9, dans laquelle ladite formulation est administrée au moins deux fois par jour.

11. Une formulation comprenant une association nutraceutique comprenant de l'astaxanthine, de la curcumine et de l'extrait sec de Cordyceps sinensis en combinaison avec des excipients et/ou des diluants appropriés.
